(19) Europäisches Patentamt
European Patent Office
Office européen des brevets

(11) EP 3 597 647 A1

(12) EUROPEAN PATENT APPLICATION
published in accordance with Art. 153(4) EPC

(43) Date of publication:
22.01.2020 Bulletin 2020/04

(21) Application number: 18768432.9

(22) Date of filing: 16.03.2018

(51) Int Cl.:
*C07D 407/04* (2006.01) *A61K 31/352* (2006.01)
*A61P 1/02* (2006.01) *A61P 1/04* (2006.01)
*A61P 7/00* (2006.01) *A61P 9/00* (2006.01)
*A61P 11/00* (2006.01) *A61P 11/04* (2006.01)
*A61P 11/08* (2006.01) *A61P 13/02* (2006.01)
*A61P 13/08* (2006.01) *A61P 13/10* (2006.01)
*A61P 13/12* (2006.01) *A61P 15/08* (2006.01)
*A61P 17/00* (2006.01) *A61P 17/02* (2006.01)
*A61P 17/18* (2006.01) *A61P 29/00* (2006.01)
*A61P 31/00* (2006.01) *A61P 31/04* (2006.01)
*A61P 39/06* (2006.01)

(86) International application number:
PCT/JP2018/010476

(87) International publication number:
WO 2018/169055 (20.09.2018 Gazette 2018/38)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 16.03.2017 JP 2017050861

(71) Applicants:
- The Kitasato Institute
Tokyo 108-8641 (JP)
- BF Agro Co., Ltd.
Tokyo 103-0027 (JP)

(72) Inventors:
- OMURA, Satoshi
Tokyo 108-8641 (JP)
- TAKAHASHI, Yoko
Tokyo 108-8641 (JP)
- NAKASHIMA, Takuji
Tokyo 108-8641 (JP)
- MATSUO, Hirotaka
Tokyo 108-8641 (JP)
- NONAKA, Kenichi
Tokyo 108-8641 (JP)
- SAKATO, Hisako
Tokyo 103-0027 (JP)

(74) Representative: J A Kemp LLP
14 South Square
Gray's Inn
London WC1R 5JJ (GB)

# (54) NOVEL POCHONIOLIDE COMPOUND AND USE THEREOF

(57) It is known that foods containing a carbohydrate, e.g., starch, in a large amount produce acrylamide upon heating at high temperatures. Some documents state that acrylamide is formed by the Maillard reaction of an amino acid, e.g., asparagine, with a reducing sugar. The purpose of the present invention is to provide a novel compound which is effective in inhibiting such foods from yielding acrylamide when treated at a high temperature. Specifically, the present invention provides a compound represented by General Formula (I).

EP 3 597 647 A1

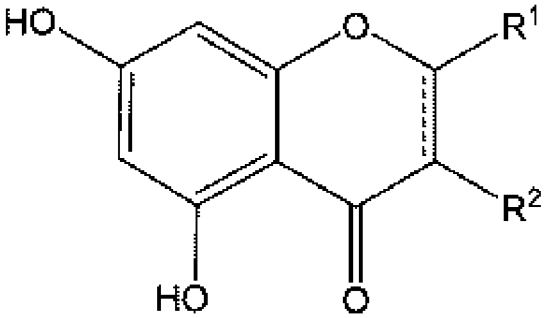

(I)

**Description**

TECHNICAL FIELD

The present invention relates to a novel pochoniolide compound, a method for producing the same, and a method for inhibiting production of acrylamide using the pochoniolide compound.

BACKGROUND ART

It is known that acrylamide is produced when heating a food containing a large quantity of carbohydrate such as starch at high temperature. Acrylamide is a compound classified as a "probable human carcinogen" by the International Agency for Research on Cancer (IARC), and health hazards caused by acrylamide consumption is an issue of concern. Some documents state that acrylamide is formed by the Maillard reaction of an amino acid such as asparagine with a reducing sugar (Non-Patent Literature 1 and Non-Patent Literature 2).

Substances that inhibit production of acrylamide have been reported. For example, a method in which asparagine is hydrolyzed into aspartic acid with the aid of asparaginase (e.g., Patent Literature 1), a method involving the use of an amino acid that inhibits production of acrylamide, such as cysteine or lysine (e.g., Patent Literature 2), a method involving the use of a reducing sugar conversion enzyme such as hexose oxidase (e.g., Patent Literature 3), a method involving the use of a divalent or trivalent cation (e.g., Patent Literature 4), a method involving the use of a thiol compound (e.g., Patent Literature 5), a method involving the use of ascorbic acid, citric acid, and the like to make use of anti-oxidant activity or pH lowering activity thereof (e.g., Patent Literature 6), and a method involving the use of an emulsifier (e.g., Patent Literature 7) are known. As substances that inhibit production of acrylamide, in addition, a sulfide compound (e.g., Patent Literature 8), luteolin (Patent Literature 9), dihydroquercetin (Patent Literature 10), flavonoid (Patent Literature 11), coriander (Patent Literature 12), trehalose (Patent Literature 13), cyclodextrin (Patent Literature 14), and xanthine and caffeine (Patent Literature 15) have been reported.

PRIOR ART DOCUMENTS

PATENT LITERATURES

Patent Literature 1: WO 2011/064280
Patent Literature 2: WO 2005/018339
Patent Literature 3: WO 2004/039174
Patent Literature 4: WO 2004/075657
Patent Literature 5: US 2005-0118322
Patent Literature 6: WO 2004/004484
Patent Literature 7: JP 2012-191928 A
Patent Literature 8: JP 2007-261983 A
Patent Literature 9: JP 2004-215559 A
Patent Literature 10: WO 2011/162250
Patent Literature 11: WO 2004/032647
Patent Literature 12: JP 2007-105015 A
Patent Literature 13: WO 2004/047559
Patent Literature 14] JP 2012-514996 A
Patent Literature 15: JP 2006-055159 A

NON-PATENT LITERATURES

Non-Patent Literature 1: Donald S. Mottram1 et al., Nature 419, 448-449, 2002
Non-Patent Literature 2: Richard H. Stadler et al., Nature 419, 449-450, 2002

SUMMARY OF THE INVENTION

OBJECTS TO BE ATTAINED BY THE INVENTION

The object of the present invention is to provide a novel compound having activity of inhibiting production of acrylamide.

MEANS FOR ATTAINING THE OBJECTS

The present invention is based on the finding described above and thus includes the following aspects.

(1) A compound represented by General Formula (I) (hereafter, referred to as "Compound (I)") or an ester derivative thereof, or a salt thereof, or a hydrate or solvate thereof:

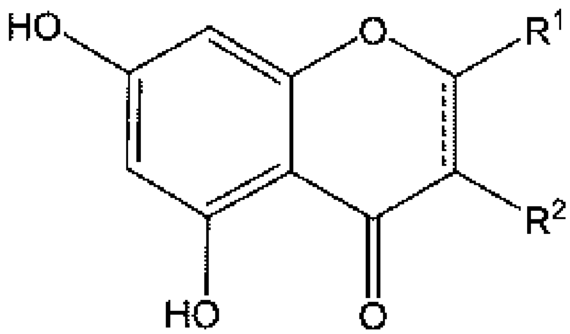

(I)

wherein a bond indicated by a solid line and a dot line represents a single bond or double bond; one of $R^1$ and $R^2$ represents a hydrogen atom or a hydroxyl group; and the other represents a group represented by Formula (X):

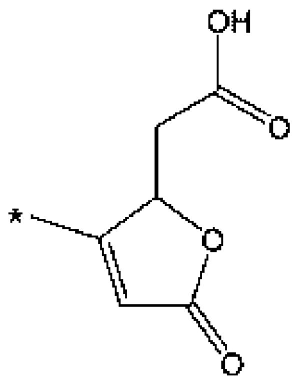

(X)

wherein * indicates a position bound to the remaining portion.

(2) The compound according to (1) or an ester derivative thereof, or a salt thereof, or a hydrate or solvate thereof, which is a compound represented by any of Formulae (Ia) to (Ih) below or an ester derivative thereof, or a salt thereof, or a hydrate or solvate thereof:

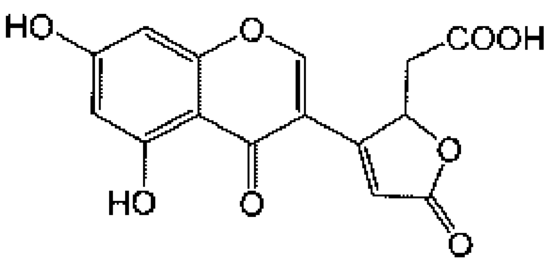

(Ia)

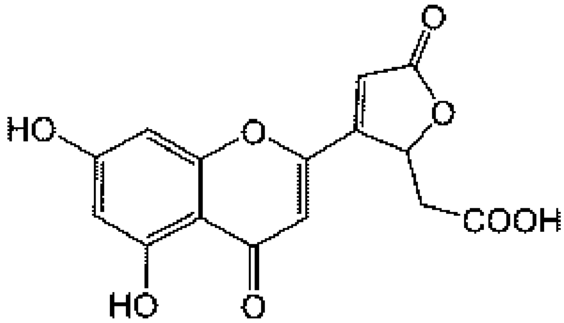

(Ib)

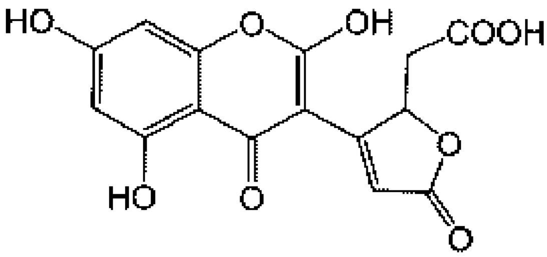

(Ic)

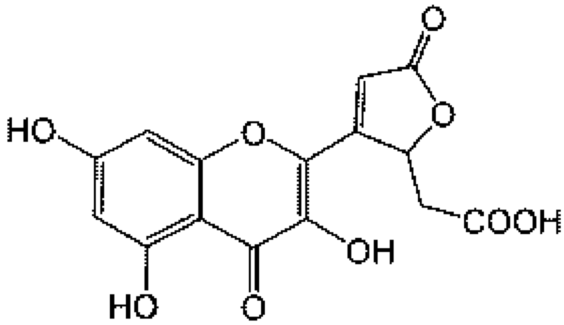

(Id)

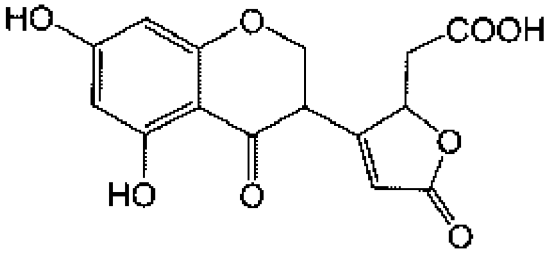

(Ie)

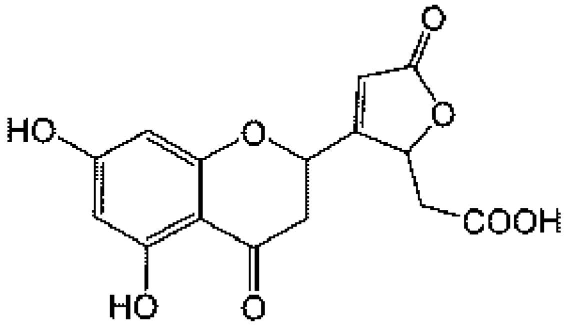

(If)

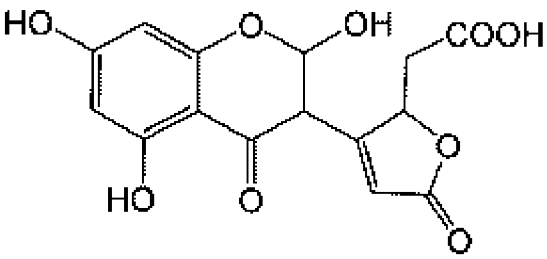

(Ig)

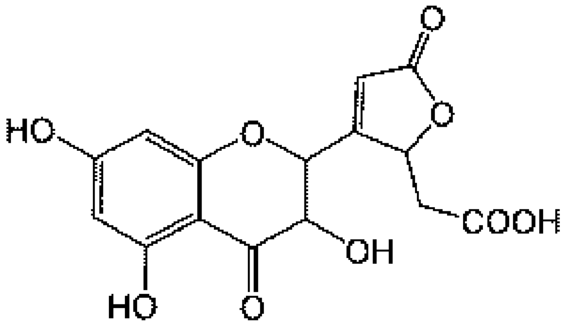

(Ih)

(3) The compound according to (2) or an ester derivative thereof, or a salt thereof, or a hydrate or solvate thereof, which is a compound represented by Formula (Ih) or an ester derivative thereof, or a salt thereof, or a hydrate or solvate thereof.

(4) A method for producing the compound according to any of (1) to (3) comprising a step of culturing a filamentous microorganism capable of producing the compound according to any of (1) to (3) in a medium to accumulate the compound in a culture broth, and a step of collecting the compound from the culture broth.
(5) The method of production according to (4), wherein the filamentous microorganism capable of producing the compound according to any of (1) to (3) is the fungal strain *Pochonia chlamydosporia var. spinulospora* FKI-7537 (Accession Number NITE BP-02441).
(6) An isolated fungal strain *Pochonia chlamydosporia var. spinulospora* FKI-7537 (Accession Number NITE BP-02441).
(7) An inhibitor of acrylamide production comprising the compound according to any of (1) to (3).
(8) A hydroxyl radical or singlet oxygen scavenger comprising the compound according to any of (1) to (3).
(9) A method for inhibiting acrylamide production comprising a step of adding the compound according to any of (1) to (3) to a food.
(10) A food additive comprising the compound according to any of (1) to (3) or an ester derivative thereof, or a salt thereof, or a hydrate or solvate thereof.
(11) A food composition comprising the compound according to any of (1) to (3) or an ester derivative thereof, or a salt thereof, or a hydrate or solvate thereof, and one or more types of food acceptable carrier, excipient, or diluent.
(12) A medicine comprising, as an active ingredient, the compound according to any of (1) to (3) or an ester derivative thereof, or a salt thereof, or a hydrate or solvate thereof.
(13) A pharmaceutical composition comprising the compound according to any of (1) to (3) or an ester derivative thereof, or a salt thereof, or a hydrate or solvate thereof, and one or more types of pharmaceutically acceptable carrier, excipient, or diluent.
(14) A method for treatment or prevention of a disease comprising a step of administering an effective amount of the compound according to any of (1) to (3) or an ester derivative thereof, or a salt thereof, or a hydrate or solvate thereof to a patient in need thereof.
(15) The compound according to any of (1) to (3) or an ester derivative thereof, or a salt thereof, or a hydrate or solvate thereof for use in treatment or prevention of a disease.
(16) Use of the compound according to any of (1) to (3) or an ester derivative thereof, or a salt thereof, or a hydrate or solvate thereof in the manufacture of a medicine or pharmaceutical composition for treatment or prevention of a disease.

The compound represented by General Formula (I) or an ester derivative thereof, or a salt thereof (a salt of the compound or ester derivative), or a hydrate or solvate thereof (a hydrate or solvate of the compound, ester derivative or salt) is occasionally referred to as "compounds of the present invention represented by General Formula (I)" herein. Also, compounds represented by Formulae (Ia) to (Ih) or ester derivatives thereof, or salts thereof (salts of the compounds or ester derivatives), or hydrates or solvates thereof (hydrates or solvates of the compounds, ester derivatives or salts) are occasionally referred to as "compounds represented by Formulae (Ia) to (Ih)" herein.
Preferably, the compound of the present invention represented by General Formula (I) satisfies any of the conditions below:

(Ia):

the bond indicated by a solid line and a dot line is a double bond;
$R^1$ represents a hydrogen atom; and
$R^2$ represents a group represented by Formula (X);

(Id):

the bond indicated by a solid line and a dot line is a double bond;
$R^1$ represents a group represented by Formula (X); and
$R^2$ represents a hydroxyl group; or

(Ih):

the bond indicated by a solid line and a dot line is a single bond;
$R^1$ represents a group represented by Formula (X); and
$R^2$ represents a hydroxyl group.

More preferably, the compound of the present invention represented by General Formula (I) satisfies the conditions below:
(Ih):

the bond indicated by a solid line and a dot line is a single bond;
$R^1$ represents a group represented by Formula (X); and
$R^2$ represents a hydroxyl group.

The compound of the present invention is preferably pochoniolide A, B, or C represented by the formula below, and more preferably pochoniolide C represented by the formula below.

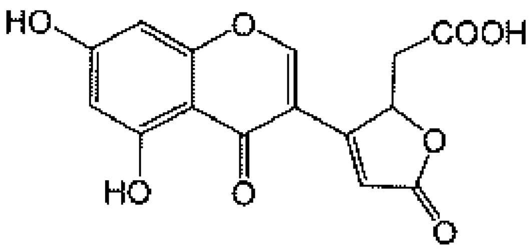

Pochoniolide A
(Ia)

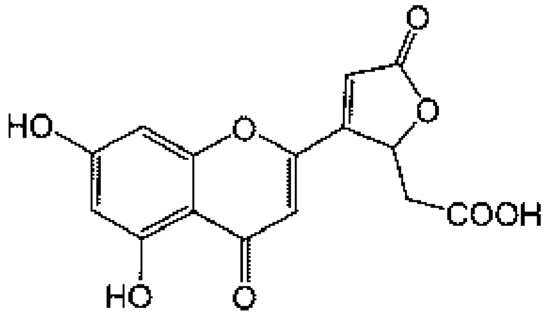

Pochoniolide B
(Id)

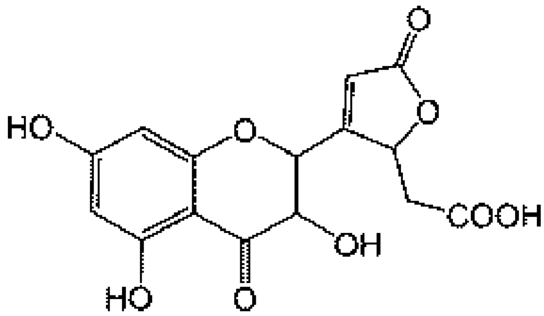

Pochoniolide C
(Ih)

In another aspect, the present invention relates to a method for producing Compound (I) represented by the formula above or an ester derivative thereof comprising: a step of culturing a filamentous microorganism capable of producing Compound (I) or an ester derivative thereof in a medium to accumulate Compound (I) represented by the formula above or an ester derivative thereof in a culture broth; and a step of collecting Compound (I) represented by the formula above or an ester derivative thereof from the culture broth.

"Pochoniolide A" herein refers to a compound having physical properties described below.

(1) Properties: yellow powder or yellow amorphous solid
(2) Molecular weight: 318
(3) Molecular formula: $C_{15}H_{10}O_8$
(4) $[M+H]^+$ determined by high-resolution mass spectrometry: theoretical value (m/z) 319.0448; measured value (m/z) 319.0461
(5) Specific rotation: $[\alpha]_D^{25}$ = 0(c = 0.1, methanol)
(6) Ultraviolet absorption maximum (in methanol) $\lambda_{max}(\varepsilon)$: 219(12149), 273(13524)
(7) Infrared absorption maximum $\nu_{max}$(KBr tablet): maximal absorption at 3437, 1709, 1627, and 1514 $cm^{-1}$
(8) $^1H$ NMR (in deuterated dimethyl sulfoxide) $\delta$ ppm: 2.37 (1H, dd, 8.8, 16.7), 3.01 (1H, m), 5.94 (1H, d, 8.0), 6.27 (1H, s), 6.44 (1H, s), 6.68 (1H, s), 8.69 (1H, s), 12.39 (OH, s)
(9) $^{13}C$ NMR (in deuterated dimethyl sulfoxide) $\delta$ ppm: 38.1, 78.7, 94.4, 99.8, 104.0, 113.2, 116.2, 157.0, 158.6, 158.7, 161.6, 165.0, 170.6, 171.8, 178.5

(10) Solubility: easily soluble in DMSO and acetone, but hardly soluble in water and methanol.

"Pochoniolide B" herein refers to a compound having physical properties described below.

(1) Properties: yellow powder or yellow amorphous solid
(2) Molecular weight: 334
(3) Molecular formula: $C_{15}H_{10}O_9$
(4) $[M+H]^+$ determined by high-resolution mass spectrometry: theoretical value (m/z) 334.0412; measured value (m/z) 334.0398
(5) Specific rotation: $[\alpha]_D^{25}$ = 0(c = 0.1, methanol)
(6) Ultraviolet absorption maximum (in methanol) $\lambda_{max}(\varepsilon)$: 215 (17045), 275 (9816)
(7) Infrared absorption maximum $\nu_{max}$(KBr tablet): maximal absorption at 3433, 1736, 1651, 1597, and 1512 $cm^{-1}$
(8) $^1H$ NMR (in deuterated dimethyl sulfoxide) $\delta$ ppm: 2.50 (1H, m), 3.06 (1H, m), 5.81 (1H, d, 7.2), 6.16 (1H, s), 6.34 (1H, s), 6.69 (1H, s)11.10 (OH, s), 12.00 (OH, s)
(9) $^{13}C$ NMR (in deuterated dimethyl sulfoxide) $\delta$ ppm: 38.0, 78.6, 93.8, 98.6, 103.8, 118.1, 138.4, 141.0, 156.3, 156.7, 161.0, 165.1, 170.7, 171.1, 175.9
(10) Solubility: easily soluble in DMSO and acetone, but hardly soluble in water and methanol.

"Pochoniolide C" herein refers to a compound having physical properties described below.

(1) Properties: pale yellow powder or pale yellow amorphous solid
(2) Molecular weight: 336
(3) Molecular formula: $C_{15}H_{12}O_9$
(4) $[M+H]^+$ determined by high-resolution mass spectrometry: theoretical value (m/z) 337.0559; measured value (m/z) 337.0549
(5) Specific rotation: $[\alpha]_D^{23}$ = -22.9(c = 0.1, methanol)
(6) Ultraviolet absorption maximum (in methanol) $\lambda_{max}(\varepsilon)$: 212 (5100), 291 (2921)
(7) Infrared absorption maximum $\nu_{max}$ (KBr tablet): maximal absorption at 3432, 1670, 1589, and 1512 $cm^{-1}$
(8) $^1H$ NMR (in deuterated dimethyl sulfoxide) $\delta$ ppm: 2.46 (1H, dd, 16.7, 9.2), 3.10 (1H, dd, 16.7, 2.7), 4.48 (1H, d, 11.5), 5.26 (1H, dd, 11.5, 2.1), 5.56 (1H, ddd, 9.2, 2.7, 2.1), 5.96 (1H, d, 1.8), 5.98 (1H, d, 1.8), 6.35 (1H, dd, 2.1, 2.1), 11.74 (OH, s)
(9) $^{13}C$ NMR (in deuterated dimethyl sulfoxide) $\delta$ ppm: 37.3, 70.6, 76.1, 79.6, 95.4, 96.7, 100.2, 117.2, 161.4, 163.3, 167.0, 167.9, 170.7, 171.2
(10) Solubility: easily soluble in DMSO and acetone, but hardly soluble in water and methanol.

The term "salt of Compound (I)" used herein refers to a salt of Compound (I) formed by binding it to an inorganic or organic base or acid. Since Compound (I) has a carboxylic acid group, Compound (I) can form a salt with: for example, alkali metal and alkaline earth metal such as lithium, sodium, potassium, magnesium, or calcium; amine such as ammonia, methylamine, dimethylamine, trimethylamine, dicyclohexylamine, tris(hydroxymethyl)aminomethane, N,N-bis(hydroxyethyl)piperadine, 2-amino-2-methyl-1-propanol, ethanolamine, N-methylglucamine, or L-glucamine; or basic amino acid such as lysine, $\delta$-hydroxylysine, or arginine. A hydrate or solvate of Compound (I) and a hydrate or solvate of a salt of Compound (I) are within the scope of the compound of the present invention. The term "Compound (I)" used in other paragraphs of the present description encompasses a salt, a hydrate or a solvate of Compound (I), and a hydrate or solvate of a salt of Compound (I), even if it is not specifically described, unless it is apparently inappropriate.

In addition to the compounds mentioned above, the compound of the present invention encompasses ester derivatives of such compounds. The "ester derivative" used herein encompasses a compound having an amide bond, in addition to a compound having an ester bond. Examples of esters include a substituted or unsubstituted lower alkyl ester, lower alkenyl ester, lower alkylamino lower alkyl ester, acylamino lower alkyl ester, acyloxy lower alkyl ester, aryl ester, aryl lower alkyl ester, amide, lower alkylamide, and hydroxylated amide. An ester is preferably a propionic acid ester or acyl ester.

The compound of the present invention occasionally has an asymmetric carbon atom. Thus, an enantiomer may be present. The compound of the present invention may be a dextrorotatory (+) or levorotatory (-) compound, or it may be an isomeric mixture, such as a racemate. The compound of the present invention encompasses a tautomer or a geometric isomer (e.g., E isomer or Z isomer), unless otherwise specified.

In an aspect, the present invention relates to an inhibitor of acrylamide production comprising Compound (I) or an ester derivative thereof, or a salt thereof, or a hydrate or solvate thereof. The inhibitor of acrylamide production of the present invention is primarily to be added to a food. In particular, it is used for inhibiting production of acrylamide produced when cooking a food with heating. The term "food" used herein means a substance that is cooked with heating

and ingested by a human or animal, and such food preferably contains a large quantity of carbohydrate such as starch.

In another aspect, the present invention relates to a food additive comprising Compound (I) or an ester derivative thereof, or a salt thereof, or a hydrate or solvate thereof. In another aspect, the present invention relates to a food composition comprising Compound (I) or an ester derivative thereof, or a salt thereof, or a hydrate or solvate thereof, and one or more types of food acceptable carrier, excipient, or diluent. In another aspect, the present invention relates to a hydroxyl radical or singlet oxygen scavenger for non-therapeutic use (e.g., for food use) comprising Compound (I) or an ester derivative thereof, or a salt thereof, or a hydrate or solvate thereof. In another aspect, the present invention relates to use of Compound (I) or an ester derivative thereof, or a salt thereof, or a hydrate or solvate thereof in the manufacture of a hydroxyl radical or singlet oxygen scavenger for non-therapeutic use (e.g., for food use).

In another aspect, the present invention relates to a hydroxyl radical or singlet oxygen scavenger comprising Compound (I) or an ester derivative thereof, or a salt thereof, or a hydrate or solvate thereof. In another aspect, the present invention relates to use of Compound (I) or an ester derivative thereof, or a salt thereof, or a hydrate or solvate thereof in the manufacture of a hydroxyl radical or singlet oxygen scavenger. According to the present aspect, the hydroxyl radical and singlet oxygen scavenger of the present invention can be applied to therapeutic use. In another aspect, the present invention relates to a medicine comprising, as an active ingredient, Compound (I) or an ester derivative thereof, or a salt thereof, or a hydrate or solvate thereof. In another aspect, the present invention relates to a pharmaceutical composition comprising Compound (I) or an ester derivative thereof, or a salt thereof, or a hydrate or solvate thereof, and one or more types of pharmaceutically acceptable carrier, excipient, or diluent. In another aspect, the present invention relates to use of Compound (I) or an ester derivative thereof, or a salt thereof, or a hydrate or solvate thereof in the manufacture of a medicine or pharmaceutical composition. The medicine or pharmaceutical composition of the present invention can be a therapeutic or preventive drug for inflammatory diseases. The term "inflammatory diseases" used herein encompasses systemic inflammatory diseases and infectious diseases, and also localized inflammatory diseases and infectious diseases. Examples of inflammatory diseases include superficial/deep skin infection, lymphangitis/lymphadenitis, mastadenitis, myelitis, amygdalitis, pneumonia, cystopyelonephritis, urethritis, gonococcal infection, syphilis, intrauterine infection, scarlatina, diphtheria, pertussis, secondary infection by external injuries, burn injuries, and surgical operations, pharyngitis/laryngitis, bronchitis, secondary infection by chronic respiratory diseases, pericoronitis, periodontitis, tetanus, cystitis, prostatitis, infectious enteritis, jaw inflammation, infectious arthritis, and gastritis.

When the medicine or pharmaceutical composition of the present invention comprises an ester derivative of Compound (I), or a salt thereof, or a hydrate or solvate thereof, such substance should be pharmaceutically acceptable. The term pharmaceutically acceptable means to be acceptable to be administered into a human or animal body in the form of a medicine. The medicine or pharmaceutical composition of the present invention is preferably a medicine or pharmaceutical composition for parenteral administration. For example, it can be in the form of an injection or pharmaceutical formulation for topical application. In the present description, a type of the medicine or pharmaceutical composition is not particularly limited, and examples of dosage forms thereof include a pharmaceutical formulation for topical application, a suspension, an ointment, a cream, a gelling agent, an inhalant, and an injection preparation (e.g., an intravenous injection preparation, a hypodermic injection preparation, an intramuscular injection preparation, or a drop). The medicine or pharmaceutical composition of the present invention may comprise a pharmaceutically acceptable carrier (a pharmaceutical additive), according to need.

This description includes part or all of the content as disclosed in the description and/or drawings of Japanese Patent Application No. 2017-050861, which is a priority document of the present application.

BRIEF DESCRIPTION OF THE DRAWINGS

Fig. 1 shows the results of $^{1}H$-$^{1}H$ COSY and $^{1}H$-$^{13}C$ HMBC correlations for pochoniolide A.

Fig. 2 shows the results of $^{1}H$-$^{1}H$ COSY and $^{1}H$-$^{13}C$ HMBC correlations for pochoniolide B.

Fig. 3 shows the results of $^{1}H$-$^{1}H$ COSY and $^{1}H$-$^{13}C$ HMBC correlations for pochoniolide C.

Fig. 4 shows a graph demonstrating the effects of pochoniolides for suppressing production of acrylamide produced by the Maillard reaction. The vertical axis shows the amount of acrylamide produced. The graph shows successively from the left to the right control group (to which an inhibitor of acrylamide production is not added), dihydroquercetin-added group (positive control group), pochoniolide A-added group, pochoniolide B-added group, and pochoniolide C-added group.

Fig. 5 shows a graph demonstrating the results of measuring hydroxyl radical scavenging activity of pochoniolides. The vertical axis shows the amount of hydroxyl radicals reacted with 5,5-dimethyl-1-pyroline-N-oxide (DMPO). The graph shows successively from the left to the right acetone-added group (control group), pochoniolide A-added group, pochoniolide B-added group, pochoniolide C-added group, and dihydroquercetin-added group (positive control group).

Fig. 6 shows a graph demonstrating the results of measuring singlet oxygen scavenging activity of pochoniolides. The vertical axis shows the electron spin density. The graph shows successively from the left to the right acetone-added group (control group), pochoniolide A-added group, pochoniolide B-added group, pochoniolide C-added group, and dihydroquercetin-added group (positive control group).

Fig. 7 shows a graph demonstrating the results of evaluation of the effects of pochoniolides for decreasing the production of acrylamide in a model food (french fries). The vertical axis shows the acrylamide concentration in a model food (ppm). The graph shows successively from the left to the right control group (to which an inhibitor of acrylamide production is not added), dihydroquercetin-added group (positive control group), pochoniolide B-added group, and pochoniolide C-added group.

## EMBODIMENTS OF THE INVENTION

The compound of the present invention (I) or an ester derivative thereof (referred to as "pochoniolides" herein) can be produced by culturing a filamentous microorganism capable of producing pochoniolides in a medium to accumulate pochoniolides in a culture broth, and collecting pochoniolides from the culture broth by separation, extraction and purification, or further chemically converting or modifying the pochoniolides. Accordingly, in an aspect, the present invention relates to a method for producing pochoniolides comprising a step of culturing a filamentous microorganism capable of producing pochoniolides in a medium to accumulate the compound in a culture broth, and a step of collecting the compound from the culture broth.

In the method for producing pochoniolides of the present invention, "filamentous microorganism capable of producing pochoniolides" is not particularly limited, provided that such microorganism is filamentous fungi capable of producing pochoniolides. Examples of fungal strains that can be used in the method for producing pochoniolides of the present invention include the fungal strains mentioned above, variants thereof, and all filamentous fungi capable of producing pochoniolides. Whether or not a microorganism of interest is "filamentous microorganism capable of producing pochoniolides" can be determined by, for example, the method described below. Specifically, 1 ml of the subject microorganism cultured in a liquid medium is inoculated into a 500-ml Erlenmeyer flask containing 100 ml of a liquid medium (pH 6.0) comprising 2.0% glucose, 0.5% yeast extract, 0.1% hipolypeptone, 0.1% agar powder, 0.05% potassium dihydrogen-phosphate, and 0.05% magnesium sulfate heptahydrate, shake culture is performed at 27°C for 3 days, 1 ml of the resulting seed culture solution is inoculated into a 500-ml Erlenmeyer flask containing 100 ml of a liquid medium (pH 6.5) comprising 3.0% soluble starch, 0.5% soy flour powder, 3.0% glycerol, 0.3% dry yeast, 0.2% potassium chloride, 0.05% calcium carbonate, 0.05% potassium dihydrogen-phosphate, 0.05% magnesium sulfate heptahydrate, and 0.03% quercetin dihydrate, and shake culture is performed at 27°C for 2 days. If pochoniolides are present in the culture broth, the subject microorganism can be determined to be capable of producing pochoniolides. Preferably, filamentous microorganism capable of producing pochoniolides is a strain of *Pochonia chlamydosporia var. spinulospora* FKI-7537 isolated from soil in Niijima, Tokyo, Japan. The subject microorganism is deposited at the International Patent Organism Depositary of the National Institute of Technology and Evaluation (122, 2-5-8 Kazusakamatari, Kisarazu-shi, Chiba, 292-0818, Japan) as of March 7, 2017 under the accession number: NITE BP-02441.

A medium used for culturing a filamentous microorganism capable of producing pochoniolides can comprise, as nutrients, substances that can be used as nutrients for filamentous fungi. Examples thereof include commercially available peptone, meat extract, corn steep liquor, cottonseed powder, peanut powder, soybean powder, yeast extract, NZ-amine, casein hydrate, nitrogen sources such as sodium nitrate, ammonium nitrate, and ammonium sulfate, carbon sources such as carbohydrates or fats (e.g., glycerin, starch, glucose, galactose, and mannose), and inorganic salts such as common salt, phosphoric acid salt, calcium carbonate, and magnesium sulfate. Such nutrients can be used alone or in combination. In addition, the medium can be supplemented with, for example, a trace amount of metal salt and, as an antifoaming agent, an animal, plant, or mineral oil, according to need. Any of such substances can be used without particular limitation, provided that such substances are useful for production of pochoniolides utilizing the production strains. All culture materials of known filamentous fungi can be used.

Filamentous microorganisms capable of producing pochoniolides can be cultured by performing shake culture in a temperature range in which production strains can grow and produce pochoniolides (e.g., 10°C to 40°C, and preferably 25°C to 30°C) for 2 to 3 days. Culture conditions can be appropriately determined in accordance with properties of pochoniolide-producing strains used with reference to the present description.

Pochoniolides can be collected by extraction with the use of a water-immiscible organic solvent such as ethyl acetate from a culture solution. In addition to the extraction method, known techniques adopted to collect lipophilic substances, such as adsorption chromatography, partition chromatography, gel filtration chromatography, scraping from thin-layer chromatography, centrifugal counter-current distribution chromatography, or high-performance liquid chromatography, may be adopted in appropriate combination, or such techniques may be repeated. Thus, the culture broth can be purified to obtain pure pochoniolides.

Also, the compound (I) of the present invention can be obtained with the use of the *Pochonia chlamydosporia*

*var. spinulospora* strain FKI-7537 as a production strain via isolation in the same manner as in the method of production and purification of pochoniolides.

An ester derivative of Compound (I) of the present invention, or a salt thereof, or a hydrate or solvate thereof can be synthesized by subjecting pochoniolides to appropriate chemical conversion or modification.

In an aspect, the present invention relates to a method for inhibiting production of acrylamide comprising a step of adding Compound (I) or an ester derivative thereof, or a salt thereof, or a hydrate or solvate thereof to a food. For example, the method of the present invention comprises a step of adding Compound (I) or the like to a food, and optionally the method further comprises a step of heating the food. Since the method of the present invention comprises the above steps, Compound (I) or the like can inhibit production of acrylamide produced upon heating at high temperature (preferably heating at 120°C or higher temperature). Compound (I) or the like can be added to a food in any manner, as long as Compound (I) or the like can sufficiently inhibit the Maillard reaction occurring between an amino acid such as asparagine and a reducing sugar such as fructose or dextrose. For example, Compound (I) or the like can be added to a food by mixing, permeation, or applying or spraying to the surface. For example, Compound (I) or the like can be added to a food by bringing Compound (I) or the like into direct contact with the food (more specifically, Compound (I) or the like are mixed with a food, Compound (I) or the like are allowed to permeate a food, or Compound (I) or the like are applied or sprayed onto the food surface). Alternatively, Compound (I) or the like can be contained in a material or vehicle (e.g., cooking oil) used for cooking a food, and the material or vehicle can be brought into contact with a food (e.g., the material or vehicle is mixed with a food, the material or vehicle is allowed to permeate a food, or the material or vehicle is applied or sprayed onto the food surface). Thus, Compound (I) or the like can be added to the food. A food is heated primarily for cooking the food (cooking with heating). An appropriate heating method can be selected in accordance with the method for cooking, and examples thereof include frying, baking, and roasting. The term "high temperature" means that temperature is over 100°C. For example, food heating temperature may be 105°C or higher, 110°C or higher, 115°C or higher, 120°C or higher, 125°C or higher, or 130°C or higher.

The inhibitor of acrylamide production, the food additive, the food composition, and the hydroxyl radical or singlet oxygen scavenger for non-therapeutic use (e.g., for food use) of the present invention can be produced by appropriate known methods. For example, the inhibitor of acrylamide production, the food additive, the food composition, and the hydroxyl radical or singlet oxygen scavenger for non-therapeutic use (e.g., for food use) of the present invention can be produced by mixing Compound (I) or the like with one or more types of food acceptable carrier, excipient or diluent, and optionally adding other food acceptable additives. A food acceptable carrier, excipient, or diluent can be appropriately selected from among various materials that are generally used in the art.

The hydroxyl radical or singlet oxygen scavenger and the pharmaceutical composition or anti-inflammatory agent of the present invention can be produced by appropriate known methods. For example, the hydroxyl radical or singlet oxygen scavenger and the pharmaceutical composition or anti-inflammatory agent of the present invention can be produced by mixing an aqueous suspension containing nanoparticles of a glucocorticosteroid compound with any ingredients in an appropriate diluent such as distilled water or purified water, adjusting the osmotic pressure and the pH level to the above levels, performing autoclave sterilization or filter sterilization in a sterile environment, and filling the sterilized container with the resultant.

In an aspect, the present invention relates to a method for treatment or prevention of a disease comprising a step of administering an effective amount of Compound (I) or the like to a patient in need thereof. In another aspect, the present invention relates to Compound (I) or the like for use in treatment or prevention of a disease. In another aspect, the present invention relates to use of Compound (I) or the like in the manufacture of a medicine or pharmaceutical composition for treatment or prevention of a disease. An example of the disease is an inflammatory disease. When Compound (I) or the like are used for therapeutic or preventive purposes, for example, the medicine or pharmaceutical composition comprising, as active ingredients, Compound (I) or the like can be administered orally or parenterally by means of an injection or droop preparation. When administering Compound (I) or the like to mammalian animals and the like, Compound (I) or the like can be administered orally in the form of a tablet, powder, granule or syrup preparation, or parenteral in the form of an injection or drop preparation. A dose varies depending on symptoms, age, sexuality, body weight, route of administration, and other conditions. For example, 0.1 to 1,000 mg is administered orally to an adult per day.

## EXAMPLES

Hereafter, the present invention is described in greater detail with reference to the following examples, although the present invention is not limited thereto. All the references cited herein are incorporated herein by reference in their entirety.

(Example 1) Mycological properties of *Pochonia chlamydosporia var. spinulospora* strain FKI-7537

The present inventors isolated the *Pochonia chlamydosporia var. spinulospora* strain FKI-7537 from soil in Niijima, Tokyo, Japan. Mycological properties of the *Pochonia chlamydosporia var. spinulospora* strain FKI-7537 are as described below.

(I) Morphological features

The strains moderately grew in potato dextrose agar medium, potato carrot agar medium, and other media, and conidia sufficiently adhered to various agar media.

The hyphae subjected to culture in the potato carrot agar medium are colorless, 2.5 to 5 $\mu$m in width, septate, and thick-walled. Conidiophores are formed on the aerial hyphae, they are not clearly distinguished from vegetative hyphae, and they are thin-walled, smooth, septate, and 2.0 to 3.0 $\mu$m in width. Phialides develop at 2 or 3 sites on top of the conidiophore. Whorls of 3 to 5 conidiogenous cells (phialides) develop on top of the conidiophore or a solitary phialide develops on the side thereof. Phialides are thin-walled, smooth, and 11.25 to 17.5 $\times$ 1.5 to 2.5 $\mu$m in size with a tapering apex of 0.25 to 0.5 $\mu$m in width. Conidia are catenated, colorless, unicellular, subglobular, elliptic in shape, thin-walled, smooth, and (2.0-) 2.5 to 3.0 $\times$ (1.75-) 2.5 to 3.0 $\mu$m in size. Muriform chlamydospores (dictyochlamydospores) develop at the tips of branches on top of the aerial hyphae, they are colorless or light brown, they are formed of a mass of irregularly elliptical cells, they are 7.5 to 15 (-28.75) $\times$ 7.5 to 12.5 $\mu$m in size, and they are smooth.

(II) Culture properties

Table 1 shows the results of visual observation when culture has been conducted in various agar media at 25°C for 7 days.

[Table 1]

| Medium | Growth on medium (colony radius) | Color of colony surface | Color of colony back surface | Soluble dye |
|---|---|---|---|---|
| Potato dextrose agar medium | Moderate (23 to 28 mm), downy, entire margin | White | Cobrless to pale yellowish white | None |
| Potato carrot agar medium | Moderate (22 to 28 mm) , downy, entire margin | White | Cobrless to pale yellowish white | None |

(III) Various physiological properties

Identification of aerobic properties or anaerobic properties

Aerobic properties

(IV) ITS rRNA gene analysis

Approximately 600 nucleotide sequences of the ITS rRNA gene were determined and compared with the sequence data of strains of the genus *Pochonia* registered and disclosed in DNA databases. As a result, the strain of interest was determined to be classified into the genus *Pochonia* and to be most closely related to the *Pochonia chlamydosporia var. spinulospora.*

(VI) Conclusion

The mycological properties of the strain are summarized as follows. Conidiophores are formed on the aerial hyphae, whorls of 3 to 5 phialides develop on top of the conidiophore, and catenated conidia develop at the tip of the phialide. In addition, muriform chlamydospores develop at the tips of branches on top of the aerial hyphae. On the basis of such results and the results of ITS rRNA gene analysis, the strain was identified to be *Pochonia chlamydosporia var. spinulospora.* This strain was deposited as the *Pochonia chlamydosporia var. spinulospora* strain FKI-7537 at the International Patent Organism Depositary of the National Institute of Technology and Evaluation (122, 2-5-8 Kazusakamatari, Kisarazu-shi, Chiba, 292-0818, Japan) as of March 7, 2017 under the accession number: NITE BP-02441.

(Example 2) Acquisition of pochoniolide A and pochoniolide B

A loopful of the *Pochonia chlamydosporia var. spinulospora* strain FKI-7537 (NITE BP-02441) subjected to slant culture was inoculated into a 500-ml Erlenmeyer flask containing 100 ml of a liquid medium (pH 6.0) comprising 2.0% glucose (Wako Pure Chemical Corporation), 0.5% yeast extract (Oriental Yeast Co., Ltd.), 0.1% hipolypeptone (Nihon Pharmaceutical Co., Ltd.), 0.1% agar powder (Shimizu Shokuhin Co., Ltd.), 0.05% potassium dihydrogen-phosphate (Kanto Chemical Co., Inc.), and 0.05% magnesium sulfate heptahydrate (Kanto Chemical Co., Inc.), and shake culture was performed at 27°C for 3 days. The resulting seed culture solution was inoculated in an amount of 1 ml into each of 60 of the 500-ml Erlenmeyer flasks containing 100 ml of a liquid medium (pH 6.5) comprising 3.0% soluble starch (Wako Pure Chemical Corporation), 0.5% soy flour powder (Kabushikikaisha Noukou Tanaka Ryuu Shouten), 3.0% glycerol (Kanto Chemical Co., Inc.), 0.3% dry yeast (JT Foods Co., Ltd.), 0.2% potassium chloride (Kanto Chemical Co., Inc.), 0.05% calcium carbonate (Kanto Chemical Co., Inc.), 0.05% potassium dihydrogen-phosphate (Kanto Chemical Co., Inc.), 0.05% magnesium sulfate heptahydrate (Kanto Chemical Co., Inc.), and 0.03% quercetin dihydrate (Wako Pure Chemical Corporation), and shake culture was performed at 27°C for 2 days.

After the completion of culture, 100 ml of ethanol was added to each of 60 of the 500-ml Erlenmeyer flasks, and the flasks were vigorously agitated for 1 hour. Subsequently, ethanol was evaporated under reduced pressure from the extract, the extract was concentrated to a volume of 1.5 liters, hydrochloric acid was added to the aqueous solution obtained to adjust a pH to 3, 1.5 liters of ethyl acetate was added thereto, the mixture was thoroughly agitated, and the ethyl acetate layer was collected. The resultant was concentrated to dryness using an evaporator, and 3.6 g of a crude product 1 was obtained. The crude product 1 was subjected to silica gel (MERCK) open column chromatography with stepwise elution (from 100:0, 98:2, 9:1, 1:1, to 0:100) in a chloroform-methanol solvent system. Thus, 600 mg of a 9:1 fraction containing pochoniolide A and pochoniolide B was obtained (a crude product 2).

The crude product 2 was subjected to separation using Purif-pack-EX, ODS-25, SIZE 60 (Shoko Science Co., Ltd.) with elution in an A: water/B: methanol system (38% B (0 to 50 min), 100% B (50 to 75 min), 18 ml/min), and a crude product fraction containing pochoniolide A and pochoniolide B was obtained. The crude product fraction was injected into an octadecyl-silylated column (Inertsil ODS-4, φ14 × 250 mm, flow rate: 10 ml/min, detection at 254 nm) via high-performance liquid chromatography and then subjected to the separation with elution with a 25% aqueous acetonitrile solution containing 0.1% formic acid. The peak at a retention time of approximately 16.7 minutes and the peak at a retention time of approximately 21.3 minutes were separated, and concentrated under reduced pressure to obtain 4.0 mg of pochoniolide A and 21.0 mg of pochoniolide B as yellow amorphous solids.

(Example 3) Acquisition of pochoniolide C

A loopful of the *Pochonia chlamydosporia var. spinulospora* strain FKI-7537 (NITE BP-02441) subjected to slant culture was inoculated into a 500-ml Erlenmeyer flask containing 100 ml of a liquid medium (pH 6.0) comprising 2.0% glucose (Wako Pure Chemical Corporation), 0.5% yeast extract (Oriental Yeast Co., Ltd.), 0.1% hipolypeptone (Nihon Pharmaceutical Co., Ltd.), 0.1% agar powder (Shimizu Shokuhin Co., Ltd.), 0.05% potassium dihydrogen-phosphate (Kanto Chemical Co., Inc.), and 0.05% magnesium sulfate heptahydrate (Kanto Chemical Co., Inc.), and shake culture was performed at 27°C for 3 days. The resulting seed culture solution was inoculated in an amount of 1 ml into each of 60 of the 500-ml Erlenmeyer flasks containing 100 ml of a liquid medium (pH 6.5) comprising 3.0% soluble starch (Wako Pure Chemical Corporation), 0.5% soy flour powder (Kabushikikaisha Noukou Tanaka Ryuu Shouten), 3.0% glycerol (Kanto Chemical Co., Inc.), 0.3% dry yeast (JT Foods Co., Ltd.), 0.2% potassium chloride (Kanto Chemical Co., Inc.), 0.05% calcium carbonate (Kanto Chemical Co., Inc.), 0.05% potassium dihydrogen-phosphate (Kanto Chemical Co., Inc.), 0.05% magnesium sulfate heptahydrate (Kanto Chemical Co., Inc.), and 0.03% Lavitol (Ametis JSC), and shake culture was performed at 27°C for 2 days.

After the completion of culture, 100 ml of ethanol was added to each of 60 of the 500-ml Erlenmeyer flasks, and the flasks were vigorously agitated for 1 hour. Subsequently, ethanol was evaporated under reduced pressure from the extract, the extract was concentrated to a volume of 1.5 liters, formic acid was added to the aqueous solution to adjust a pH to 3, 1.5 liters of ethyl acetate was added thereto, the mixture was thoroughly agitated, and the ethyl acetate layer was collected. The resultant was concentrated to dryness using an evaporator, and 1.9 g of a crude product 1 was obtained. The crude product 1 was subjected to silica gel (MERCK) open column chromatography with stepwise elution (from 100:0, 98:2, 9:1, 1:1, to 0:100) in a chloroform-methanol solvent system. Thus, 607 mg of a 9:1 fraction containing pochoniolide C was obtained (a crude product 2).

The crude product 2 was subjected to separation using Purif-pack-EX, ODS-25, SIZE 60 (Shoko Science Co., Ltd.) with elution in an A: water/B: methanol system (0% B (0 to 60 min), 100% B (60 to 70 min), 20 ml/min), and a crude product fraction 3 containing pochoniolide C was obtained. The crude product fraction 3 (58 mg) was subjected to separation using an octadecyl-silylated column (YMC-Triart C-18, φ10 × 250 mm, flow rate: 5 ml/min, detection at 254 nm) via high-performance liquid chromatography with elution with a 20% aqueous acetonitrile solution containing 0.1%

formic acid. The peak at a retention time of approximately 15.0 minutes was separated, and concentrated under reduced pressure to obtain 20.0 mg of pochoniolide C as a pale yellow amorphous solid.

Physicochemical properties of the pochoniolides were determined as follows. Physicochemical properties of pochoniolide A were as follows.

(1) Properties: yellow powder or yellow amorphous solid
(2) Molecular weight: 318
(3) Molecular formula: $C_{15}H_{10}O_8$
(4) $[M+H]^+$ determined by high-resolution mass spectrometry: theoretical value (m/z) 319.0448; measured value (m/z) 319.0461
(5) Specific rotation: $[\alpha]_D^{25}$ = 0(c = 0.1, methanol)
(6) Ultraviolet absorption maximum (in methanol) $\lambda_{max}(\varepsilon)$: 219(12149), 273(13524)
(7) Infrared absorption maximum $\nu_{max}$(KBr tablet): maximal absorption at 3437, 1709, 1627, and 1514 $cm^{-1}$
(8) Proton nuclear magnetic resonance spectrum: Table 2 shows chemical shifts in deuterated dimethyl sulfoxide (ppm). (In the table, s indicates a singlet, d indicates a doublet, m indicates a multiplet, and H indicates the number of protons.)
(9) Carbon nuclear magnetic resonance spectrum: Table 2 shows chemical shifts in deuterated dimethyl sulfoxide (ppm).
(10) Fig. 1 shows the results of $^1H$-$^1H$ COSY and $^1H$-$^{13}C$ HMBC correlations for pochoniolide A.
(11) Solubility: easily soluble in DMSO and acetone, but hardly soluble in water and methanol.

[Table 2]

| **Position** | **$\delta_C$, mult** | **$\delta_H$ (int., mult., *J* in Hz)** |
|---|---|---|
| 2 | 158.6, CH | 8.69(1H, s) |
| 3 | 113.2, C | |
| 4 | 178.5, C | |
| 4a | 104.0, C | |
| 5 | 161.6, C | |
| 5-OH | | 12.39 |
| 6 | 99.8, CH | 6.27 (1H, s) |
| 7 | 165.0, C | |
| 8 | 94.4, CH | 6.44 (1H, s) |
| 8a | 157.0, C | |
| 1' | 171.8, C | |
| 2' | 116.2, CH | 6.68 (1H, s) |
| 3' | 158.7, C | |
| 4' | 78.7, CH | 5.94 (1H, d, 8.0) |
| 5' | 38.1, $CH_2$ | a : 2.37 (1H, dd, 8.8, 16.7) |
| | | b : 3.01 (1H, m) |
| 6' | 170.6, C | |

Physicochemical properties of pochoniolide B were as follows.

(1) Properties: yellow powder or yellow amorphous solid
(2) Molecular weight: 334
(3) Molecular formula: $C_{15}H_{10}O_9$
(4) $[M+H]^+$ determined by high-resolution mass spectrometry: theoretical value (m/z) 334.0412; measured value (m/z) 334.0398
(5) Specific rotation: $[\alpha]_D^{25}$ = 0 (c = 0.1, methanol)
(6) Ultraviolet absorption maximum (in methanol) $\lambda_{max}(\varepsilon)$: 215 (17045), 275 (9816)
(7) Infrared absorption maximum $\nu_{max}$(KBr tablet): maximal absorption at 3433, 1736, 1651, 1597, and 1512 $cm^{-1}$
(8) Proton nuclear magnetic resonance spectrum: Table 3 shows chemical shifts in deuterated dimethyl sulfoxide (ppm). (In the table, s indicates a singlet, d indicates a doublet, m indicates a multiplet, and H indicates the number of protons.)

(9) Carbon nuclear magnetic resonance spectrum: Table 3 shows chemical shifts in deuterated dimethyl sulfoxide (ppm).
(10) Fig. 2 shows the results of $^1H$-$^1H$ COSY and $^1H$-$^{13}C$ HMBC correlations for pochoniolide B.
(11) Solubility: easily soluble in DMSO and acetone, but hardly soluble in water and methanol.

[Table 3]

| Position | $\delta_C$, mult | $\delta_H$ (int., mult., *J* in Hz) |
|---|---|---|
| 2 | 138.4, C | |
| 3 | 141.0, C | |
| 4 | 175.9, C | |
| 4a | 103.8, C | |
| 5 | 161.0, C | |
| 5-OH | | 12.0 (1H) |
| 6 | 98.6, CH | 6.16 (1H, s) |
| 7 | 165.1, C | |
| 7-OH | | 11.1 (1H) |
| 8 | 93.8, CH | 6.34 (1H, s) |
| 8a | 156.3, C | |
| 1' | 171.1,C | |
| 2' | 118.1, CH | 6.69 (1H, s) |
| 3' | 156.7, C | |
| 4' | 78.6, CH | 5.81 (1H, d, 7.2) |
| 5' | 38.0, $CH_2$ | a: 2.50 (1H, m) |
| | | b: 3.06 (1H, m) |
| 6' | 170.7, C | |

Physicochemical properties ofpochoniolide C were as follows.

(1) Properties: pale yellow powder or pale yellow amorphous solid
(2) Molecular weight: 336
(3) Molecular formula: $C_{15}H_{12}O_9$
(4) $[M+H]^+$ determined by high-resolution mass spectrometry: theoretical value (m/z) 337.0559; measured value (m/z) 337.0549
(5) Specific rotation: $[\alpha]_D^{23.3}$ = -22.9(c = 0.1, methanol)
(6) Ultraviolet absorption maximum (in methanol) $\lambda_{max}(\varepsilon)$: 215 (17045), 275 (9816)
(7) Infrared absorption maximum $\nu_{max}$(KBr tablet): maximal absorption at 3432, 1670, 1589, and 1512 $cm^{-1}$
(8) Proton nuclear magnetic resonance spectrum: Table 4 shows chemical shifts in deuterated dimethyl sulfoxide (ppm). (In the table, s indicates a singlet, d indicates a doublet, m indicates a multiplet, and H indicates the number of protons.)
(9) Carbon nuclear magnetic resonance spectrum: Table 4 shows chemical shifts in deuterated dimethyl sulfoxide (ppm).
(10) Fig. 3 shows the results of $^1H$-$^1H$ COSY and $^1H$-$^{13}C$ HMBC correlations for pochoniolide C.
(11) Solubility: easily soluble in DMSO and acetone, but hardly soluble in water and methanol.

[Table 4]

| Position | $\delta_C$, mult | $\delta_H$ (int., mult., *J* in Hz) |
|---|---|---|
| **2** | **76.1** | **5.26 (1H, dd, 11.5, 2.1)** |
| **3** | **70.6** | |
| **4** | **195.9** | |
| **4a** | **100.3** | |
| **5** | **163.3** | |

(continued)

| Position | $\delta_C$, mult | $\delta_H$ (int., mult., *J* in Hz) |
|---|---|---|
| 5-OH | | 11.7 (1H, br s) |
| 6 | 96.7 | 5.96 (1H, d, 1.8) |
| 7 | 167.0 | |
| 8 | 95.4 | 5.98 (1H, d, 1.8) |
| 8a | 161.4 | |
| 1' | 171.2 | |
| 2' | 117.2 | 6.35 (1H, dd, 2.1, 2.1) |
| 3' | 167.9 | |
| 4' | 79.6 | 5.56 (1H, ddd, 9.2, 2.7, 2.1) |
| 5' | 37.3 | 2.46(1H, dd, 16.7, 9.2) |
| | | 3.10 (1H, dd, 16.7, 2.7) |
| 6' | 170.6 | |

In the past, no compounds corresponding to pochoniolides A, B, and C with various physicochemical properties as described above have been reported. Accordingly, pochoniolides A, B, and C are considered to be novel substances.

(Example 3) Influence on acrylamide produced by Maillard reaction using a mixture of asparagine and glucose

An aqueous solution (0.25 ml) of 100 mg/ml glucose (Wako Pure Chemical Corporation) treated with Chelex 100 (Bio-Rad Laboratories, Inc.) and an aqueous solution (0.5 ml) of 50 mg/ml asparagine (Wako Pure Chemical Corporation) treated with Chelex 100 (Bio-Rad Laboratories, Inc.) were introduced into a 1.5-ml microtube. Pochoniolides A, B, and C dissolved in methanol to a final concentration of 6 mg/ml and dihydroquercetin (final concentration 10% methanol) as a positive control were added, and the total amount of the solution was adjusted to 1.0 ml with the addition of distilled water. After the resultant was heated in an autoclave at 121°C for 20 minutes, the amount of acrylamide produced was measured with the use of Morinaga acrylamide EIA kit (Morinaga Institute of Biological Science, Inc.) in accordance with the protocols.

Fig. 4 shows the results of the test performed with the use of Morinaga acrylamide EIA kit. When the sample was not treated, acrylamide was produced at 51 ng/ml. When dihydroquercetin was treated at 6 mg/ml (final concentration), in contrast, the amount of acrylamide produced was 28 ng/ml. When pochoniolides A, B, and C were treated at 6 mg/ml (final concentration), the amounts of acrylamide produced were 53 ng/ml, 9 ng/ml, and 18 ng/ml, respectively.

The results obtained above demonstrate that pochoniolides B and C can suppress production of acrylamide induced by the Maillard reaction in a mixture of glucose and asparagine more efficiently than dihydroquercetin.

(Example 4) Activity of pochoniolides A, B and C for scavenging various active oxygen species

Pochoniolides A, B, and C were evaluated in terms of hydroxyl radical (·OH) and singlet oxygen ($^1O_2$) scavenging activity using dihydroquercetin as a positive control. Active oxygen species were measured in accordance with the method described in Yusuke Katsuda et al., 2015, PLOS ONE, doi:10.1371/journal.pone.0138394.

In the case of the hydroxyl radical (·OH) scavenging activity test, compounds were dissolved in acetone at 0.5 mg/ml. In the case of the singlet oxygen ($^1O_2$) scavenging activity test, compounds were dissolved in acetone at 0.25 mg/ml. Thus, hydroxyl radical (·OH) scavenging activity and singlet oxygen ($^1O_2$) scavenging activity were evaluated. The results are shown in Figs. 5 and 6. The hydroxyl radical (·OH) scavenging activity level was lower in the order of pochoniolide C, pochoniolide B, pochoniolide A, and dihydroquercetin. The singlet oxygen ($^1O_2$) scavenging activity level was lower in the order of pochoniolide B, pochoniolide A, pochoniolide C, and dihydroquercetin.

The results demonstrated above indicate that pochoniolides A, B, and C have hydroxyl radical (·OH) or singlet oxygen ($^1O_2$) scavenging activity superior to that of dihydroquercetin.

(Example 5) Cytotoxicity test of pochoniolides *in vitro*

Cytotoxicity of the pochoniolides of the present invention on various cells was tested. Human cervical cancer-derived HeLaS3 cells, human lung cancer-derived A549 cells, human lung cancer-derived H1299 cells (not expressing p53), human colon adenocarcinoma HT29 cells, human pancreatic cancer Panc1 cells, human acute T cell leukemia-derived Jurkat cells, human promyelocyte leukemia HL60 cells, and human acute monocytic leukemia THP-1 cells were

subjected to evaluation of cytotoxicity by measuring cell viability using WST-8 (Dojindo Laboratories). The cells that had reached 80% confluence were counted using a hemocytometer, and a cell suspension was prepared. The cell suspension was dispensed into each well of a 96-well microplate at 100 μl/well using multichannel pipettes. Only a medium was added to a blank (background) well in an amount of 100 μl. HeLaS3 cells, A549 cells, H1299 cells, HT29 cells, and Panc1 cells were cultured for 24 hours in a $CO_2$-incubator, Jurkat cells, HL60 cells, and THP-1 cells were cultured for 1 to 2 hours in a $CO_2$-incubator. Solutions of the compounds diluted 3-fold from 10 mM to 100 μM in methanol or dimethyl sulfoxide were prepared, and the resulting solutions were then added at 1 μl/well (final concentration: 100 μM to 1 μM). After culture was performed for 2 days, 10 μl of the WST-8 reagent was introduced into each well, culture was performed for 30 to 60 minutes, and the absorbance at 460 nm was measured. The cell viability was determined in accordance with the following equation.

$$\text{Cell viability (\%)} = [(As - Ab)/(Ac - Ab)] \times 100$$

As: Absorbance of sample (a well containing cells, pochoniolide A, B, or C, and a WST-8 solution)
Ac: Absorbance of control (a well containing cells and a WST-8 solution without pochoniolide A, B, or C)
Ab: Absorbance of blank (a well containing a medium and a WST-8 solution without cells)

As a result of the cytotoxicity tests of pochoniolide A, B, or C, pochoniolide A, B, or C was determined to show no toxicity on all the cells at 100 μM. Thus, pochoniolide A, B, and C are considered to be safe substances that are not toxic to animal cells.

(Example 6) Antibacterial activity test of pochoniolides A, B, and C *in vitro*

Antibacterial activity of the pochoniolides A, B, and C of the present invention was tested in the manner described below. 20 μl each of a solution of 5 mg/ml pochoniolide A, B, or C in methanol was spotted on filter paper discs (diameter: 8 mm, Advantech Co., Ltd.), the filter paper discs were air-dried for a given period of time, and solvents were then removed. Thereafter, the filter paper discs were allowed to adhere to an agar plate containing microorganisms, such as *Bacillus subtilis* ATCC 6633, *Micrococcus luteus* ATCC 9341, *Escherichia coli* NIHJKB213, *Xanthomonas campestris pv. oryzae* KB 88, *Candida albicans* KF1, and *Mucor racemosus* IFO4581. *Bacillus subtilis* ATCC 6633, *Micrococcus luteus* ATCC 9341, and *Escherichia coli* NIHJKB213 were cultured at 37°C for 24 hours. *Xanthomonas campestris pv. oryzae* KB 88, *Candida albicans* KF1, and *Mucor racemosus* IFO4581 were cultured at 27°C for 48 hours. Thereafter, a diameter of a growth-inhibiting circle formed in the vicinity of filter paper discs was determined.

As a result of the antibacterial activity test, pochoniolides A, B, and C were found to exert no antibacterial activity on all the tested microorganisms. Thus, pochoniolides A, B, and C are considered to be safe substances that would not adversely affect resident microorganisms.

(Example 7) Effects ofpochoniolides B and C for decreasing acrylamide in model food

Pochoniolides B and C were evaluated in terms of effects of decreasing acrylamide in a model food (french fries) using dihydroquercetin as a positive control.

Commercially available frozen french fries (raw) were thawed and a portion of 10 g was separated. The portion of 10 g was ground in a mortar and formed into individual homogeneous portions of 1 g each, followed by forming. The test compounds were dissolved in a small amount of ethanol to a final concentration of 1%. The ethanol solution was homogeneously applied to the formed potatoes. The potatoes were fried in oil at 170 to 200 degrees for 1 minute, and measurement of the acrylamide concentration was requested to Morinaga Institute of Biological Science, Inc. The results are shown in Fig. 7. While the concentration of acrylamide produced was 7 ppm with the addition of control sample, the concentration of acrylamide produced was 4 ppm with the addition of dihydroquercetin as a positive control. No effects of pochoniolide B for decreasing the amount of acrylamide production were observed. The concentration of acrylamide produced was 2 ppm in the group to which pochoniolide C was added. That is, the effects of pochoniolide C for decreasing acrylamide were found to be stronger than those of dihydroquercetin.

The results demonstrated above indicate that pochoniolide C has effects of decreasing acrylamide superior to those of dihydroquercetin.

All publications, patents, and patent applications cited herein are incorporated herein by reference in their entirety.

## Claims

1. A compound represented by General Formula (I) or an ester derivative thereof, or a salt thereof, or a hydrate or solvate thereof:

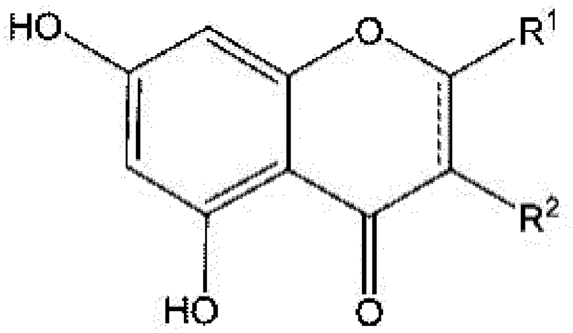

(I)

wherein a bond indicated by a solid line and a dot line represents a single bond or double bond; one of $R^1$ and $R^2$ represents a hydrogen atom or a hydroxyl group; and the other represents a group represented by Formula (X):

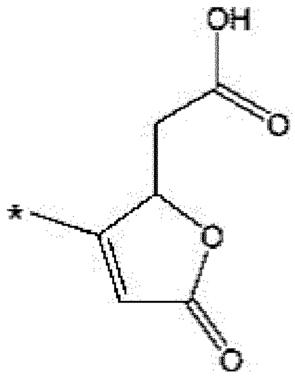

(X)

wherein * indicates a position bound to the remaining portion.

2. The compound according to Claim 1 or an ester derivative thereof, or a salt thereof, or a hydrate or solvate thereof, which is a compound represented by any of Formulae (Ia) to (Ih) below or an ester derivative thereof, or a salt thereof, or a hydrate or solvate thereof:

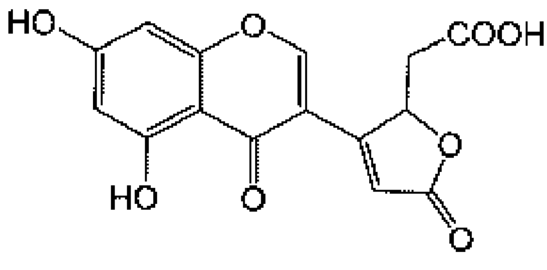

(Ia)

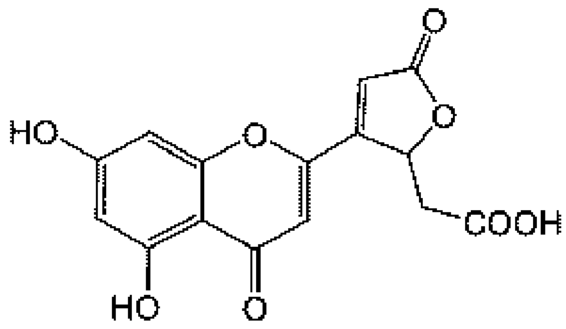

(Ib)

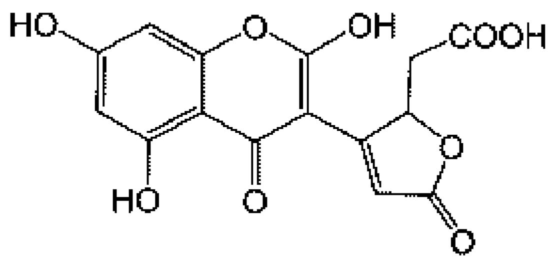

(Ic)

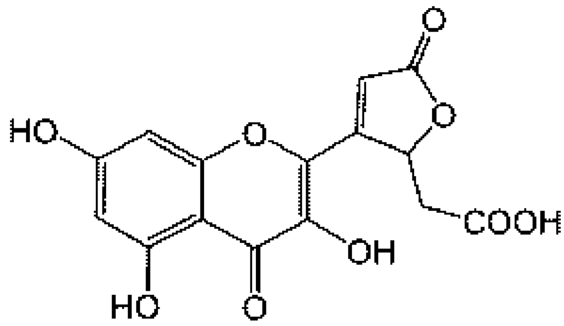

(Id)

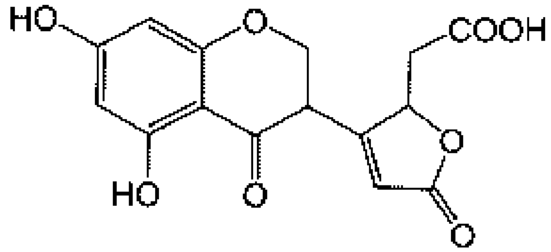

(Ie)

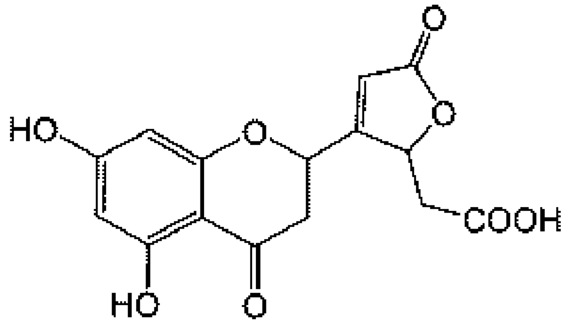

(If)

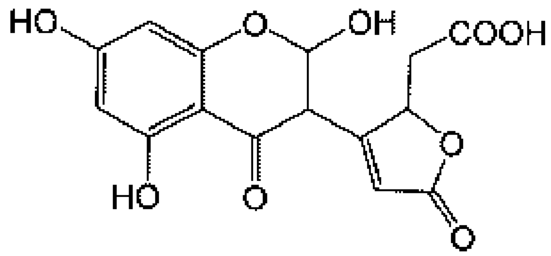

(Ig)

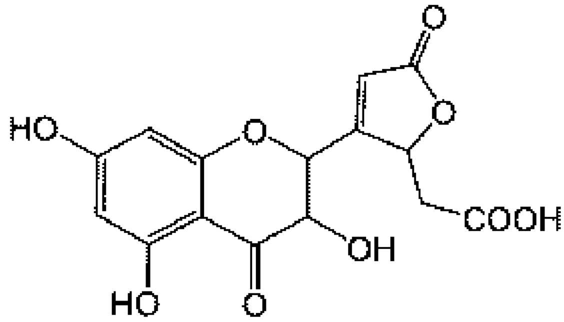

(Ih)

**3.** The compound according to Claim 2 or an ester derivative thereof, or a salt thereof, or a hydrate or solvate thereof, which is a compound represented by Formula (Ih) or an ester derivative thereof, or a salt thereof, or a hydrate or solvate thereof.

**4.** A method for producing the compound according to any one of Claims 1 to 3 comprising a step of culturing a filamentous microorganism capable of producing the compound according to any one of Claims 1 to 3 in a medium to accumulate the compound in a culture broth, and a step of collecting the compound from the culture broth.

**5.** The method of production according to Claim 4, wherein the filamentous microorganism capable of producing the compound according to any one of Claims 1 to 3 is the fungal strain *Pochonia chlamydosporia var. spinulospora* FKI-7537 (Accession Number NITE BP-02441).

**6.** An isolated fungal strain *Pochonia chlamydosporia var. spinulospora* FKI-7537 (Accession Number NITE BP-02441).

**7.** An inhibitor of acrylamide production comprising the compound according to any one of Claims 1 to 3.

**8.** A hydroxyl radical or singlet oxygen scavenger comprising the compound according to any one of Claims 1 to 3.

**9.** A method for inhibiting acrylamide production comprising a step of adding the compound according to any one of

Claims 1 to 3 to a food.

Fig. 1

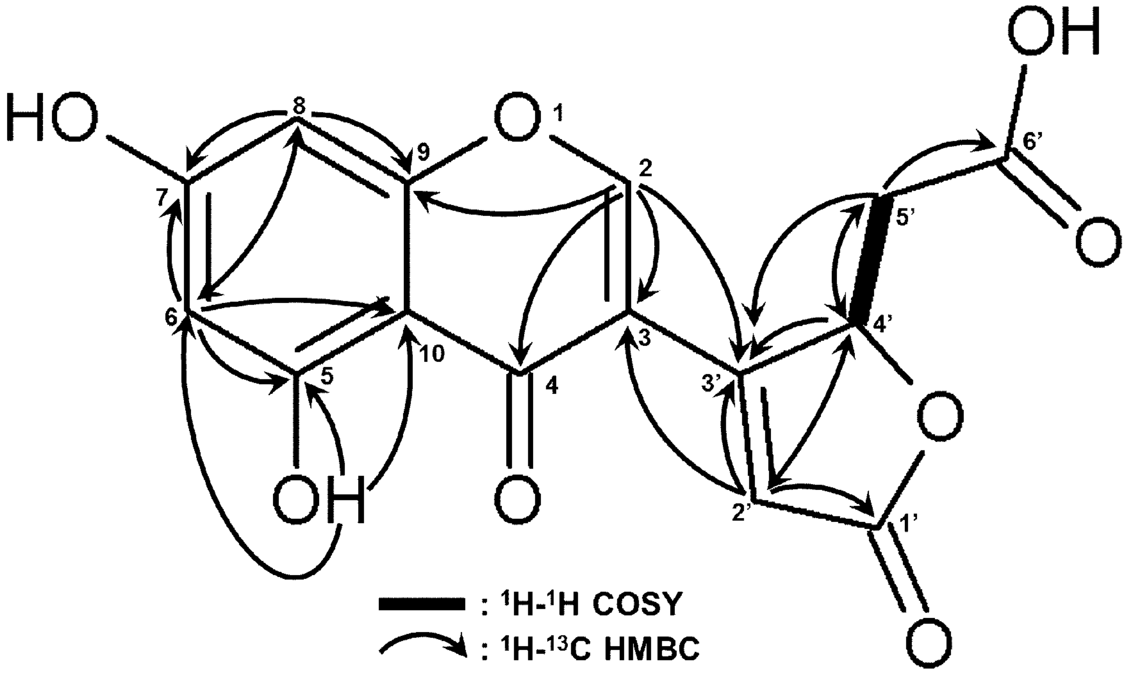

Fig. 2

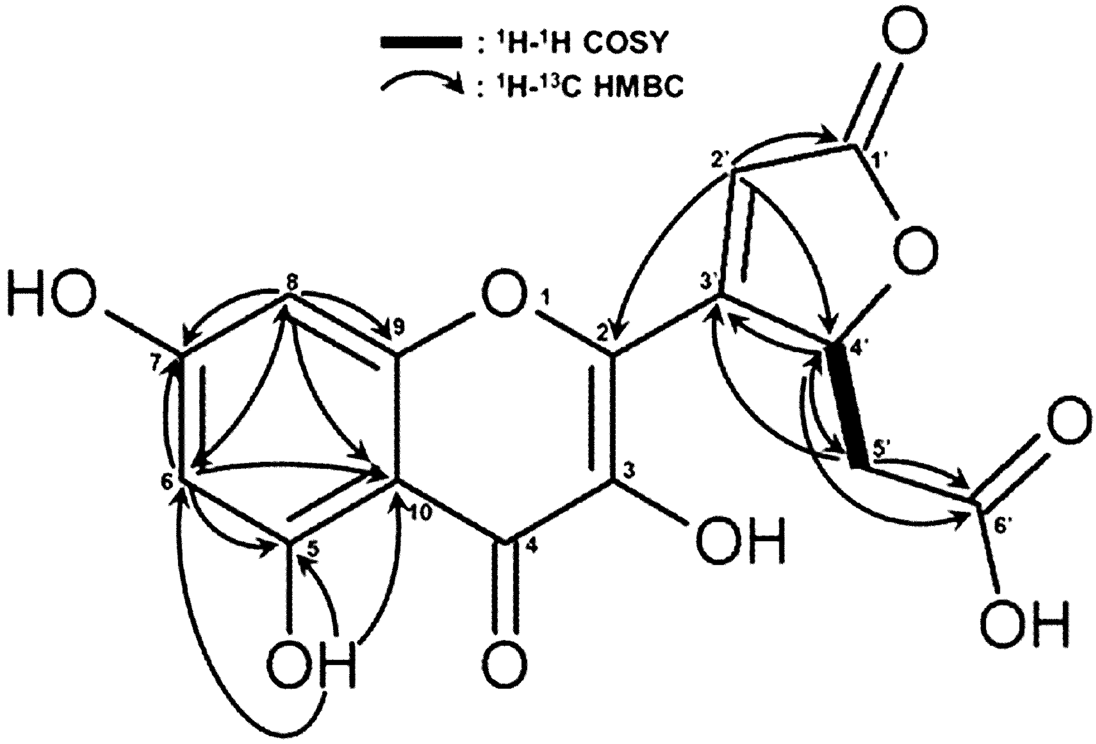

Fig. 3

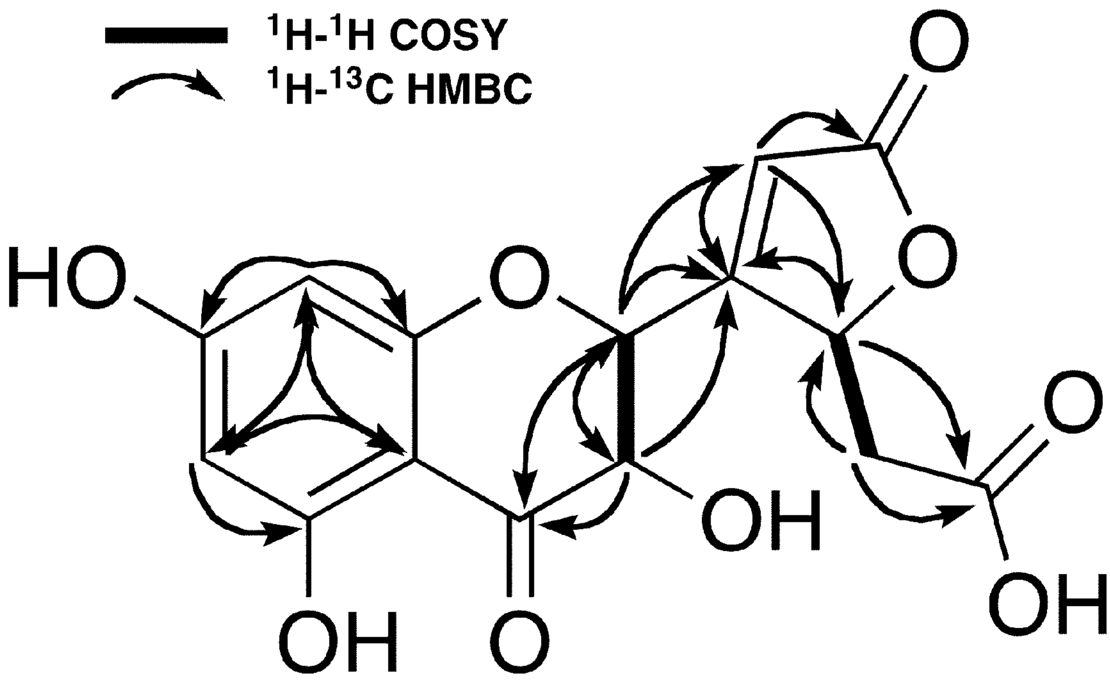

Fig. 4

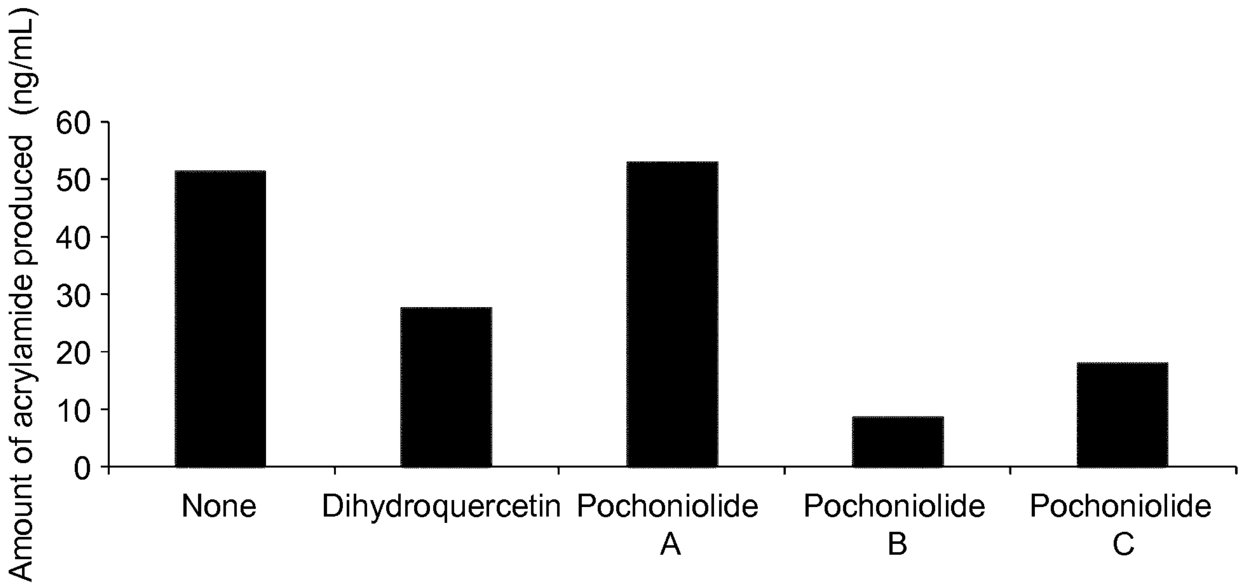
Amount of acrylamide produced (ng/mL)
60
50
40
30
20
10
0
None
Dihydroquercetin
Pochoniolide A
Pochoniolide B
Pochoniolide C

Fig. 5

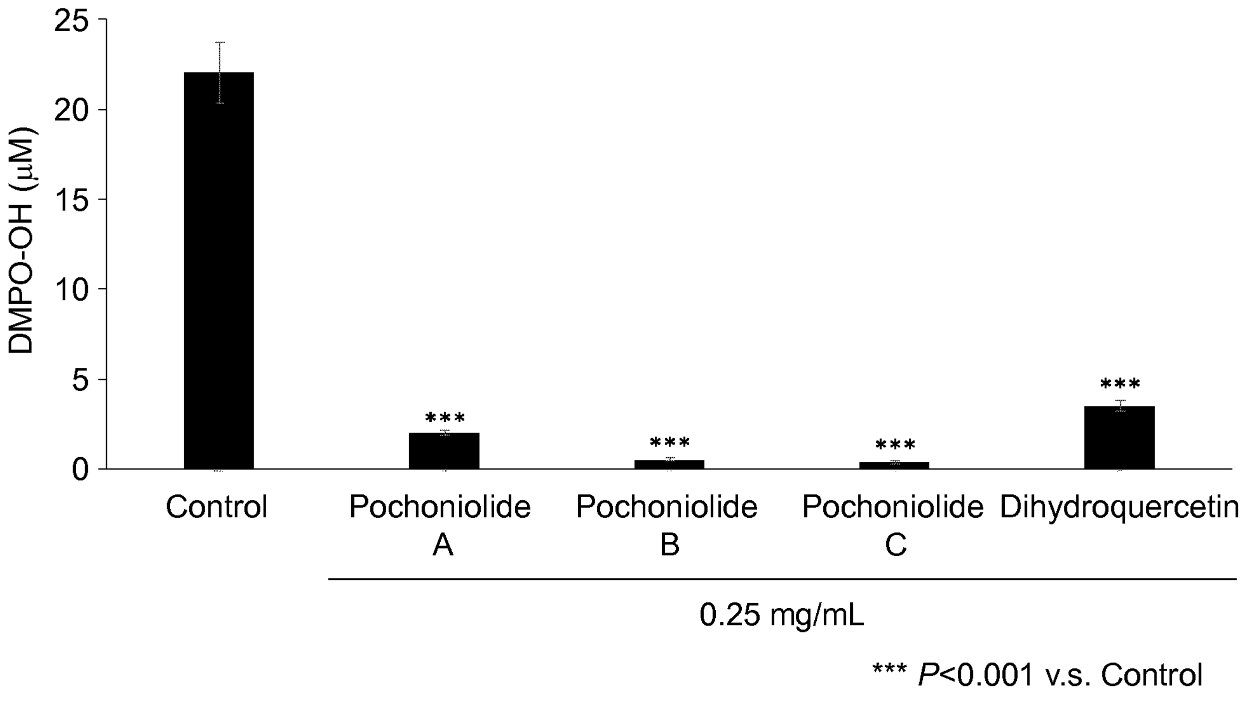
25
20
15
10
5
0
DMPO-OH (μM)
***
***
***
***
Control
Pochoniolide A
Pochoniolide B
Pochoniolide C
Dihydroquercetin
0.25 mg/mL
*** *P*<0.001 v.s. Control

Fig. 6

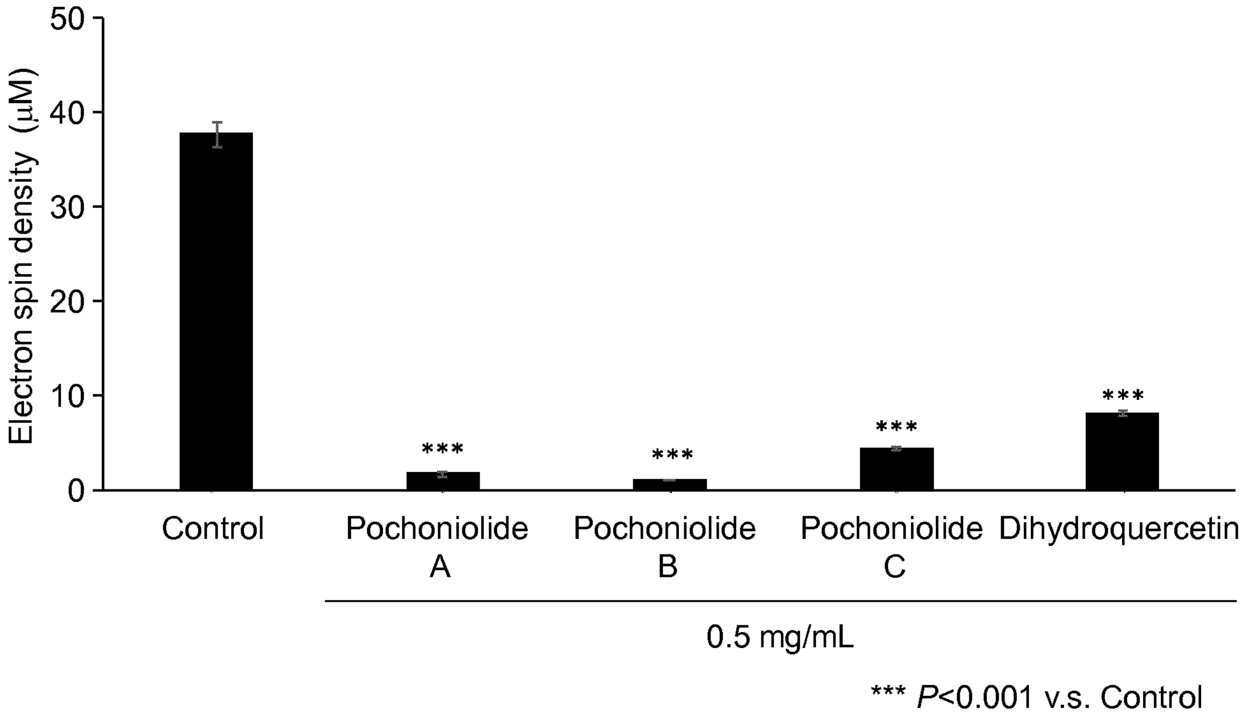
50
40
30
20
10
0
Electron spin density (μM)
***
***
***
***
Control
Pochoniolide A
Pochoniolide B
Pochoniolide C
Dihydroquercetin
0.5 mg/mL
*** P<0.001 v.s. Control

Fig. 7

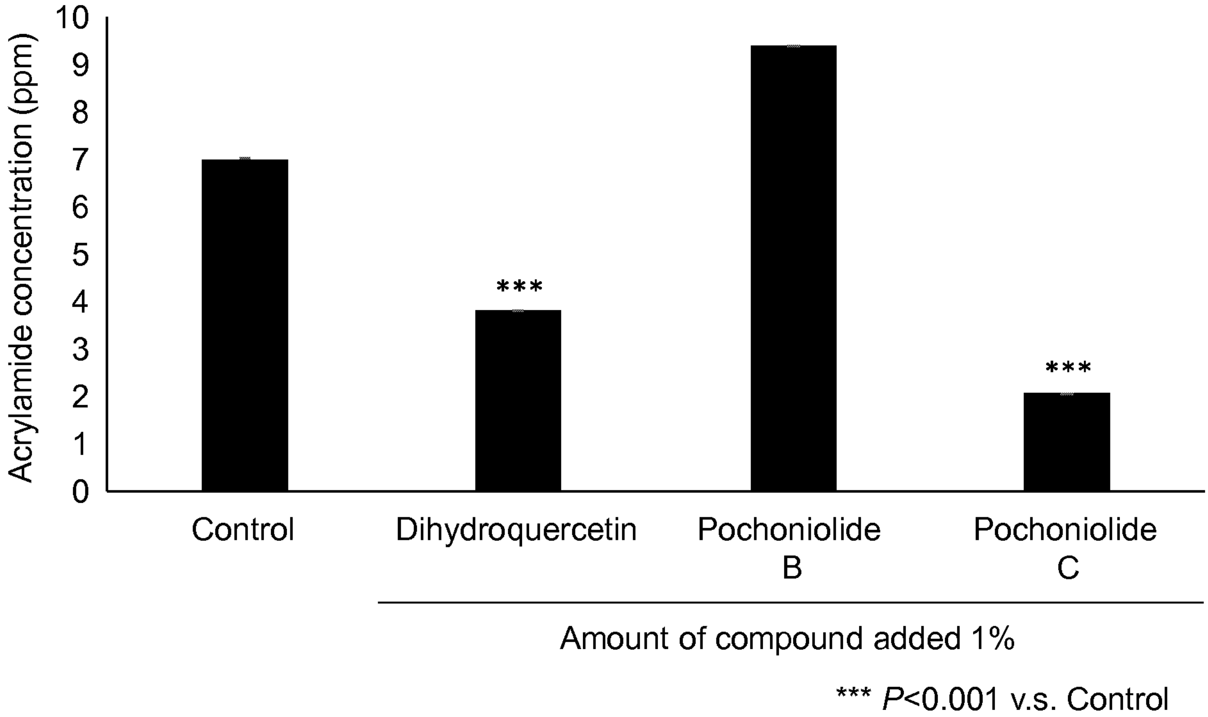
10
9
8
7
6
5
4
3
2
1
0
Acrylamide concentration (ppm)
***
***
Control
Dihydroquercetin
Pochoniolide B
Pochoniolide C
Amount of compound added 1%
*** P<0.001 v.s. Control

## INTERNATIONAL SEARCH REPORT

International application No.
PCT/JP2018/010476

A. CLASSIFICATION OF SUBJECT MATTER
See extra sheet

According to International Patent Classification (IPC) or to both national classification and IPC

B. FIELDS SEARCHED

Minimum documentation searched (classification system followed by classification symbols)
Int.Cl. C07D407/04, A61K31/352, A61P1/02, A61P1/04, A61P7/00, A61P9/00, A61P11/00, A61P11/04, A61P11/08, A61P13/02, A61P13/08, A61P13/10, A61P13/12, A61P15/08, A61P17/00, A61P17/02, A61P17/18, A61P29/00, A61P31/00, A61P31/04, A61P39/06, C12P17/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | 官野怜ほか, 糸状菌 Pochonia sp. FKI-7537 株培養液抽出物から得られた新規化合物 pochoniolide A および B について, 日本農芸化学会 2017 年度大会講演要旨集 [online], lecture no. 3C13a03, 05 March 2017, [retrieval date 25 May 2018], entire text <URL:https:katosei.jsbba.or.jp/MeetingofJSBBA2017.pdf>, non-official translation (KANNO, Rei, et al. "Regarding novel compounds, pochoniolide A and B, obtained from extract of filamentous fungi Pochonia sp. FKI-7537 strain culture medium", Lecture abstracts of 2017 annual meeting of the Japan Society for Bioscience, Biotechnology and Agrochemistry) | 1-6, 8<br>7, 9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 May 2018 (28.05.2018) | 05 June 2018 (05.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/010476

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2004/032647 A1 (SLK FOUNDATION) 22 April 2004, page 1, line 26 to page 3, line 19 & FI 20021807 A & AU 2003264663 A | 7, 9 |
| P, X | 官野怜ほか, 糸状菌 Pochonia sp. FKI-7537 株より得られた新規抗酸化物質 pochoniolides について, 環境微生物系学会合同大会プログラム集, p. 138, 29 August 2017, [retrieval date 25 May 2018], internet <URL:http://environmental-microbiology.org/2017/pdfs/要旨集/要旨集_ポスター6.pdf>, non-official translation (KANNO, Rei, et al. "Regarding novel antioxidant, pochoniolides, obtained from filamentous fungi Pochonia sp. KFI-7537 strain", Programs of joint conference of the Societies for Environmental Microbiology) | 1-6, 8 |
| A | NONAKA, K., et al. , "Three new Pochonia taxa (Clavicipi taceae) from soils in Japan", Mycologia, 2013, vol. 105, no. 5, pp. 1202-1218 | 1-9 |

Form PCT/ISA/210 (continuation of second sheet) (January 2015)

INTERNATIONAL SEARCH REPORT

International application No.
PCT/JP2018/010476

CLASSIFICATION OF SUBJECT MATTER
C07D407/04(2006.01)i, A61K31/352(2006.01)i, A61P1/02(2006.01)i, A61P1/04(2006.01)i, A61P7/00(2006.01)i, A61P9/00(2006.01)i, A61P11/00(2006.01)i, A61P11/04(2006.01)i, A61P11/08(2006.01)i, A61P13/02(2006.01)i, A61P13/08(2006.01)i, A61P13/10(2006.01)i, A61P13/12(2006.01)i, A61P15/08(2006.01)i, A61P17/00(2006.01)i, A61P17/02(2006.01)i, A61P17/18(2006.01)i, A61P29/00(2006.01)i, A61P31/00(2006.01)i, A61P31/04(2006.01)i, A61P39/06(2006.01)i, C12P17/06(2006.01)n

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011064280 A
- WO 2005018339 A
- WO 2004039174 A
- WO 2004075657 A
- US 20050118322 A
- WO 2004004484 A
- JP 2012191928 A
- JP 2007261983 A
- JP 2004215559 A
- WO 2011162250 A
- WO 2004032647 A
- JP 2007105015 A
- WO 2004047559 A
- JP 2012514996 A
- JP 2006055159 A
- JP 2017050861 A


### Non-patent literature cited in the description


- **DONALD S. MOTTRAM1 et al.** *Nature,* 2002, vol. 419, 448-449
- **RICHARD H. STADLER et al.** *Nature,* 2002, vol. 419, 449-450
- **YUSUKE KATSUDA et al.** *PLOS ONE,* 2015